(19) Europäisches Patentamt
European Patent Office
Office européen des brevets

(11) **EP 3 177 252 B1**

## (12) EUROPÄISCHE PATENTSCHRIFT

(45) Veröffentlichungstag und Bekanntmachung des Hinweises auf die Patenterteilung:
**07.06.2023 Patentblatt 2023/23**

(21) Anmeldenummer: **15744903.4**

(22) Anmeldetag: **29.07.2015**

(51) Internationale Patentklassifikation (IPC):
***A61L 15/00*** *(2006.01)*

(52) Gemeinsame Patentklassifikation (CPC):
(C-Sets verfügbar)
**A61F 13/538; A61F 13/537; A61L 15/24;
A61L 15/26; A61L 15/42** (Forts.)

(86) Internationale Anmeldenummer:
**PCT/EP2015/067365**

(87) Internationale Veröffentlichungsnummer:
**WO 2016/020236 (11.02.2016 Gazette 2016/06)**

(54) **AUFNAHME- UND VERTEILUNGSSCHICHT FÜR EINE AUFZUNEHMENDE FLÜSSIGKEIT UND DARAUS HERGESTELLTE PRODUKTE**

ABSORBING AND DISTRIBUTING LAYER FOR A LIQUID TO BE ABSORBED AND PRODUCTS PRODUCED THEREFROM

COUCHE D'ABSORPTION ET DE RÉPARTITION POUR UN LIQUIDE À ABSORBER ET PRODUITS FABRIQUÉS À PARTIR DE CETTE COUCHE

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **05.08.2014 DE 102014011372**

(43) Veröffentlichungstag der Anmeldung:
**14.06.2017 Patentblatt 2017/24**

(73) Patentinhaber: **Fitesa Germany GmbH
31224 Peine (DE)**

(72) Erfinder:
• **HARTL, Helmut
38124 Braunschweig (DE)**
• **NOVARINO, Elena
31224 Peine (DE)**
• **SIEBNER, Harald
38122 Braunschweig (DE)**

(74) Vertreter: **dompatent von Kreisler Selting Werner -
Partnerschaft von Patent- und Rechtsanwälten
mbB
Deichmannhaus am Dom
Bahnhofsvorplatz 1
50667 Köln (DE)**

(56) Entgegenhaltungen:
**EP-A1- 2 692 321      EP-A2- 0 547 498
WO-A1-93/09745        WO-A1-2013/152809
US-A1- 2005 227 564   US-A1- 2010 310 845**

(52) Gemeinsame Patentklassifikation (CPC): (Forts.)

C-Sets
**A61L 15/24, C08L 23/06;**
**A61L 15/24, C08L 23/12;**
**A61L 15/26, C08L 67/02;**
**A61L 15/26, C08L 67/04**

**Beschreibung**

Die vorliegende Erfindung betrifft eine Aufnahme- und Verteilungsschicht für eine aufzunehmende Flüssigkeit, ein Flächengebilde zumindest bestehend aus einer entsprechenden Aufnahme- und Verteilungsschicht, einen Hygieneartikel zumindest bestehend aus einer entsprechenden Aufnahme- und Verteilungsschicht, , sowie der Verwendung der entsprechenden Aufnahme- und Verteilungsschicht.

Aufnahme- und Verteilungsschichten sind dazu geeignet, Flüssigkeiten aufzunehmen, zu verteilen und die Flüssigkeit gegebenenfalls in weitere Schichten beispielsweise an einen Saugkern zu transportieren. Insbesondere werden Aufnahme- und Verteilungsschichten in Hygieneprodukten wie beispielsweise Windeln, Inkontinenzeinlagen, Slipeinlagen, Damenbinden oder Kosmetikpads verwendet. Diese Hygieneprodukte sind dazu bestimmt, Körperflüssigkeiten aufzunehmen und nach der Aufnahme zurückzuhalten. Die Aufnahme- und Verteilungsschichten dienen in Hygieneartikeln unter anderem dazu, die häufig punktuell und/oder schlagartig austretenden Körperflüssigkeiten möglichst rasch aufzunehmen, zu verteilen und an weitere Absorptionsschichten abzugeben, damit ein trockenes Gefühl beim Benutzer der Hygieneartikel beibehalten werden kann.

Aufnahme- und Verteilungsschichten für Flüssigkeiten sind aus dem Stand der Technik bekannt.

In US 5,314,743 werden Aufnahme- und Verteilungsschichten beschrieben, die geformte Fasern mit mindestens einer Kerbe umfassen und zur Absorption und zum Transport von Flüssigkeiten dienen. Die beschriebenen Aufnahme- und Verteilungsschichten werden als Zwischenschicht zwischen eine fluiddurchlässige körperseitige Einlage und einer fluidundurchlässigen Außenhülle verwendet.

EP 0 547 498 A1 beschreibt ebenfalls eine Aufnahme- und Verteilungsschicht. Stapelfasern werden hier verwendet, um diese Schicht zu bilden.

US 2010/310845 A1 offenbart ein flüssigkeitsdurchlässiges, strukturiertes Faservlies mit thermisch stabilen Fasern, die unter Verwendung von Wärme thermisch miteinander verbunden sind und ein Basissubstrat erzeugen, das thermisch stabil ist.

Häufig tritt bei Hygieneartikeln jedoch der Fall auf, dass eine sogenannte Rücknässung, auch als rewet oder wetback bezeichnet, des Hygieneartikels auftritt. Von Rücknässung spricht man bei einem Hygieneprodukt dann, wenn aus der mit Flüssigkeit beladenen Lage, zum Beispiel dem Saugkern, wieder Flüssigkeit in Richtung Körper abgegeben wird. Ziel moderner Hygieneprodukte ist es, dass die Haut des Benutzers möglichst trocken bleibt, das heißt, möglichst wenig Flüssigkeit in Richtung Körper zurückgeführt wird.

Durch die Verwendung von modernen Saugkernen, die Kunststoffe, sog. Superabsorber, umfassen, die in der Lage sind, ein Vielfaches ihres Eigengewichts an Flüssigkeiten aufzusaugen, kann zwar ein Großteil der Flüssigkeit im Saugkörper gut festgehalten werden, allerdings kann sich der Saugkörper feucht anfühlen.

Zudem wird bei Hygieneartikeln wie beispielsweise Windeln, Inkontinenzeinlagen, Slipeinlagen oder Damenbinden häufig ein großer Druck auf die Saugkerne ausgeübt, wenn beispielsweise die den Hygieneartikel tragende Person sitzt beziehungsweise sich setzt und dadurch ein großer Teil des Körpergewichts auf den Saugkern wirkt. Hierbei besteht die Möglichkeit, dass bereits aufgenommene Flüssigkeit aus dem Saugkern gedrückt und in Richtung Körper abgegeben wird.

Als Folge der Rücknässung eines Hygieneartikels können wegen des ständig vorhandenen feuchten Mikroklimas Hautirritationen beim Träger auftreten.

Um dem Träger von Hygieneprodukten ein möglichst angenehmes Tragegefühl zu ermöglichen, sind neben einer geringen Rücknässung zwei weitere Eigenschaften besonders wichtig. Zum einen ist wichtig, dass die obere Schicht des Hygieneproduktes, die mit der Haut in Kontakt steht, eine hinreichende Weichheit besitzt, die ein angenehmes Gefühl auf der Haut sicherstellt. Zum anderen sollen die Hygieneprodukte möglichst klein und dünn sein, damit sie während des Tragens vom Träger selbst aber auch von Dritten möglichst nicht wahrgenommen werden und/oder nicht zu einer Behinderung oder Beeinträchtigung der tragenden Person führen.

Üblicherweise wird das Hautgefühl beziehungsweise die Weichheit der Hygieneprodukte durch eine flüssigkeitsdurchlässige Decklage, auch topsheet genannt, eingestellt, die sich auf der Aufnahme- und Verteilungsschicht befindet und zur Kontaktierung der Haut der tragenden Personen gedacht ist. Durch die Verwendung dieser Decklage kann zwar ein angenehmes Gefühl auf der Haut sicherstellen, jedoch führt die zusätzliche Schicht dazu, dass die Gesamtdicke des Hygieneproduktes zunimmt, was im Vergleich zu dünneren Hygieneprodukten die Trageeigenschaften des Hygieneproduktes verschlechtert.

Es besteht daher eine ständige Nachfrage nach verbesserten Hygieneprodukten, die sehr dünn sind und - verglichen mit bereits bekannten Hygieneprodukten - das Tragegefühl, die Absorptionsfähigkeit und die Rücknässung zumindest beibehalten oder verbessern.

Dabei ist die Verwendung von Aufnahme- und Verteilungsschichten nicht nur auf die Verwendung in Hygieneartikeln wie beispielsweise Kosmetikpads, Windeln, Inkontinenzeinlagen, Slipeinlagen oder Damenbinden beschränkt. Aufnahme- und Verteilungsschichten mit verbesserten Eigenschaften lassen sich überall einsetzen wo eine schnelle Aufnahme und Verteilung von Flüssigkeiten nötig ist, beispielsweise in Wischtüchern, als Isolationsmaterial, als Filtermaterial oder als Umhüllungsmaterial für Saugkerne.

Aufgabe der vorliegenden Erfindung ist es daher, eine Aufnahme- und Verteilungsschicht zur Verfügung zu stellen, die zumindest eine, vorzugsweise jedoch mehrere, der nachfolgenden Eigenschaften aufweist:

- gutes Aufnahmevermögen von Flüssigkeiten, insbesondere auch unter Druck,
- gutes Verteilungsvermögen von Flüssigkeiten in der Schicht,
- geringe Rücknässung,
- Verbesserung der Trageeigenschaften beziehungsweise Verbesserung von sonstigen Eigenschaften bei gleichbleibenden Trageeigenschaften,
- geringe Produktions- und/oder Materialkosten,
- geringe Dichte der Schicht,
- geringe Dicke der Schicht,
- geringes Gewicht der Schicht,
- geringer Materialverbrauch bei der Herstellung der Schicht.

Diese Aufgabe wird erfindungsgemäß gelöst durch eine Aufnahme- und Verteilungsschicht für eine aufzunehmende Flüssigkeit mit den Merkmalen des Anspruchs 1, mit einem Flächengebilde mit den Merkmalen des Anspruchs 9, mit einem Hygieneartikel mit den Merkmalen des Anspruchs 10, sowie der Verwendung gemäß Anspruch 11. Weitere Aspekte der vorliegenden Erfindung ergeben sich aus der nachfolgenden Beschreibung, insbesondere den beschriebenen Beispielen sowie den beigefügten Patentansprüchen und Figuren. Dabei können alle Kombinationen wie auch nur einzelne

Kombinationen zwischen den nachfolgenden Merkmalen der Aufnahme- und Verteilungsschicht zusammen genutzt werden.

Weiterhin ist es jeweils auch vorgesehen und möglich einzelne oder mehrere Merkmale der Aufnahme- und Verteilungsschicht beliebig zu kombinieren.

Es wird eine Aufnahme- und Verteilungsschicht für eine aufzunehmende Flüssigkeit vorgeschlagen, die wenigstens ein Vlies umfasst, das aus trilobalen Fasern besteht, wobei das Vlies ein Spinnvlies ist. Eine erfindungsgemäße Aufnahme- und Verteilungsschicht kann somit aus einem Vlies bestehen. Es ist auch möglich, dass sie mehrere erfindungsgemäße Vliese aus trilobalen Fasern umfasst. Eine weitere Ausgestaltung sieht vor, dass die Aufnahme- und Verteilungsschicht eine oder mehrere Lagen aus Vlies aufweist, wobei zumindest eine der Lagen, bevorzugt zumindest zwei Lagen, aus trilobalen Fasern bestehen. Vorzugsweise besteht jede Lage aus trilobalen Fasern. In einer besonders bevorzugten Ausführungsform weist die erfindungsgemäße Aufnahme- und Verteilungsschicht nur Fasern mit einer trilobalen Form auf. Fasern, die einen anderen Querschnitt aufweisen, sind in dieser Ausführungsform nicht enthalten. Die Fasern können gleich wie auch unterschiedlich sein, aus dem gleichen Material, insbesondere thermoplastischen Material, insbesondere gesponnen sein. Wird im Folgenden die Aufnahme- und Verteilungsschicht beschrieben, so ist hierunter in erster Linie das erfindungsgemäße Vlies beziehungsweise in bestimmten Ausführungsformen die erfindungsgemäßen Vliese zu verstehen. Umfasst die erfindungsgemäße Aufnahme- und Verteilungsschicht nicht nur ein erfindungsgemäßes Vlies sondern weitere Vliese, so ist die ganze Schicht, die alle Vliese umfasst, entsprechend zu verstehen.

Die Aufnahme- und Verteilungsschicht ist erfindungsgemäß so ausgestaltet, dass die Aufnahme- und Verteilungsschicht eine Rücknässung gemessen nach dem EDENA Standard Test: WSP 80.10 (05) von 0,01 g bis 0,50 g, vorzugsweise 0,05 g bis 0,3 g, besonders bevorzugt 0,1 g bis 0,25 g aufweist.

Hierdurch wird vermieden, dass bereits von der Aufnahme- und Verteilungsschicht aufgenommene und gegebenenfalls weitergeleitete Flüssigkeit bei Druck auf die Aufnahme- und Verteilungsschicht wieder freigegeben wird. Insbesondere beim Einsatz der Aufnahme- und Verteilungsschicht in Hygieneprodukte kann hierdurch ein angenehmes Tragegefühl beim Träger erreicht werden und ein feuchten Mikroklima, das zu Hautirritationen beim Träger führen kann, wird vermieden.

Darüber hinaus ist es bevorzugt, dass die Aufnahme- und Verteilungsschicht hydrophil ist.

Der Begriff "hydrophil" bezeichnet ein Material, das einen Wasser-in-Luft-Kontaktwinkel von weniger als 90 Grad hat. Der Wasser-in-Luft-Kontaktwinkel wird nach der Beschreibung in dem Buch "Absorbency", herausgegeben von P. K. Chatterjee (Elsevier, New York, 1985), bestimmt. Die Hydrophilie der Aufnahme- und Verteilungsschicht kann dabei auf unterschiedliche Weisen eingestellt werden. Beispielsweise können inhärent hydrophile trilobale Fasern verwendet werden. Unter "inhärent" hydrophilen Fasern werden Fasern verstanden, die frei von Oberflächenmodifizierungen oder -behandlungen, beispielsweise frei von oberflächenaktiven Mitteln, Spinnzusätzen, Glanzmitteln usw., hydrophil sind. Auch ist es möglich die Hydrophilie der Fasern durch eine entsprechende Oberflächenmodifizierung oder -behandlungen einzustellen.

In einer weiteren Ausführungsform ist es daher bevorzugt, dass die Aufnahme- und Verteilungsschicht mittels eines, dem Fachmann geläufigen Ausrüstverfahrens beispielsweise Tränken, Besprühen, Foulardieren oder Kissroll-Auftrag mit einer hydrophilen Ausrüstung versehen werden. Insbesondere sind Aufnahme- und Verteilungsschichten bevorzugt, die mit einer Tensid-Lösung behandelt wurden. Grundsätzlich sind alle Arten von Tensiden dafür geeignet, also anionische, kationische, und ebenso nicht-ionische oder zwitterionische Tenside. Beispielsweise kann es sich bei der Tensid-Lösung um eine Lösung handeln, die Stantex® der Firma Cognis, Düsseldorf (Deutschland), oder Silastol PHP26 von Schill & Seilacher, Böblingen (Deutschland), umfasst und/oder um eine Lösung, die Rizinusethoxylate und/oder PEG-Diester enthält.

Gemäß einer bevorzugten Ausgestaltung der vorliegenden Erfindung besitzt die Aufnahme- und Verteilungsschicht eine Durchdringungszeit gemessen nach dem EDENA Standard Test: WSP 70.3 (05) von weniger als 4 Sekunden.

Aufnahme- und Verteilungsschichten mit einer geringen Durchdringungszeit haben den Vorteil, dass Flüssigkeiten schnell aufgenommen und weitergeleitet werden können. Insbesondere bei der Verwendung der Aufnahme- und Verteilungsschichten in Hygieneprodukten, wie beispielsweise Windeln, ist es wichtig, dass schlagartig auftretende Flüssigkeitsmengen, wie sie beispielsweise beim Urinieren entstehen, schnellstmöglich aufgenommen und weitergeleitet werden. Hierdurch kann beispielsweise der Tragekomfort verbessert und ein seitliches Austreten der Flüssigkeit vermieden werden.

Üblicherweise handelt es sich bei der Durchdringungszeit und der Rücknässung um einander gegenläufige Eigenschaften. Schichten, die Flüssigkeiten schnell aufnehmen, geben diese in der Regel auch schnell wieder ab und führen zu einer hohen Rücknässung, insbesondere wenn Druck auf die Schicht ausgeübt wird. Im vorliegenden Fall hat es sich überraschenderweise gezeigt, dass Aufnahme- und Verteilungsschicht Flüssigkeiten sehr gut aufnehmen und eine geringe Durchdringungszeit aufweisen, gleichzeitig aber auch eine sehr geringe Rücknässung aufweisen, da ein Rückschlagen der Flüssigkeit verhindert wird. Diese Eigenschaft kann mit der Form der trilobalen Fasern erklärt werden. Sobald Druck auf die Aufnahme- und Verteilungsschichten und somit auch auf die einzelnen Fasern ausgeübt wird, werden die Fasern vermutlich flachgedrückt und hierdurch breiter, was zu einer geringeren Rücknässung führt. Dieses Verhalten ist mit üblichen Rundfasern nicht zu erzielen.

In einer bevorzugten Ausführungsform der Aufnahme- und Verteilungsschicht ist die Aufnahme- und Verteilungsschicht so ausgebildet, dass die trilobalen Fasern der Aufnahme- und Verteilungsschicht aus einem Polyolefin, insbesondere Polypropylen oder Polyethylen, ihren Copolymeren oder Mischungen damit, überwiegend bestehen. Neben einem Polymer weisen Fasern üblicherweise noch Pigmente, Stabilisierer insbesondere gegen thermischen Kettenabbau wie gegebenenfalls auch sonstige Batches auf. Deren Bestandteile betragen üblicherweise aber weniger als 5 Gew.-% der Fasern, mit Ausnahme bei Verwendung von Füllstoffen. Deren Anteil kann durchaus höher sein. Ebenfalls einsetzbare thermoplastische Polymere sind z.B. Polylactide, deren Copolymere oder deren Mischungen. Ganz besonders bevorzugt ist die Verwendung von Polypropylen in Form von Ziegler-Natta-PP oder Metallocene-PP, aber auch in Form von Mischungen von diesen.

Bei der Verwendung von Polypropylen als Faserstoff ist die Verwendung von isotaktischem Polypropylen bevorzugt.

Durch die Verwendung der oben aufgeführten Materialien kann beispielsweise die Oberflächenbeschaffenheit der Aufnahme- und Verteilungsschicht so eingestellt werden, dass die Aufnahme- und Verteilungsschicht eine hinreichende Weichheit besitzt und ein angenehmes Gefühl auf der Haut sichergestellt wird.

Üblicherweise ist besonders die Aufnahme- und Verteilungsschicht mittels Air-Through-Bonding oder mittels Resin-Bonding verfestigt. Diese Verfestigungsverfahren verleihen den Vliesen eine gewisse Formstabilität, aber auch eine hohe Steifheit, die dem Tragekomfort je nach Ausprägung bzw. Einsatzzweck entgegenwirken kann. Vor allem für Air-Through-Bonding ist die Verwendung von gegebenenfalls teuren Bicofasern - leicht schmelzende Außenhülle mit thermisch stabilem Faserkern - oftmals vorgegeben. Insbesondere das Air-Trough-Bonding Verfahren wird angewendet, um die an sich im Vlies dicht gepackten Rundfasern "aufzulockern". Damit wird die erforderliche Porosität des Vlieses erzeugt, um eine möglichst gleichmäßige Verteilung der eindringenden Flüssigkeit zu gewährleisten. Zum anderen verbessert dieses die an sich geringe Widerstandskraft oder Knittererholung, auch "resilience" bezeichnet, von Vliesen aus runden Fasern. Das an sich wirtschaftlich günstigste Verfestigungsverfahren - thermisches Kalandrieren - ist hier mit einigen Nachteilen versehen. Die an sich schon dicht gepackten Fasern würden zwischen geheizten Walzen noch weiter verdichtet werden. Dies würde jedoch die erforderlichen Vlieseigenschaften komplett zerstören.

Es hat sich nun weiterhin überraschend gezeigt, dass Vliese aus Trilobalfasern auf Grund ihres Faserquerschnitts, ganz besonders wenn sie auf Polypropylen und/oder Polyethylen basieren, sehr wohl thermokalandriert werden können, ohne dass die resultierenden Vliese auch nur annähernd die erforderliche Funktionalität verlieren. Dies ist ein ökonomisch außerordentlich begrüßenswerter Befund, da sich nun bessere Vliesfunktionen billiger und schneller herstellen lassen.

Abhängig von dem Verwendungszweck der Aufnahme- und Verteilungsschicht sind unterschiedliche Flächengewichte der Aufnahme- und Verteilungsschicht sinnvoll. Bei Hygieneartikeln sind dünne und/oder leichte Aufnahme- und Verteilungsschichten bevorzugt, da hierdurch der Tragekomfort für den Träger verbessert werden kann. Bei der Verwendung von Aufnahme- und Verteilungsschichten in beispielsweise Tüchern oder Kosmetikpads kann es jedoch auch bevorzugt sein, dass die Aufnahme- und Verteilungsschicht dicker beziehungsweise schwerer ist, da hierdurch üblicherweise die Flüssigkeitsaufnahme der Aufnahme- und Verteilungsschicht erhöht werden kann.

Die Aufnahme- und Verteilungsschicht weist erfindungsgemäß ein Flächengewicht nach DIN EN 29073-1 von 5 g/m$^2$ bis 60 g/m$^2$, vorzugsweise von 7 g/m$^2$ bis 40 g/m$^2$, weiter bevorzugt 8 g/m$^2$ bis 20 g/m$^2$, insbesondere 10 g/m$^2$ bis 17 g/m$^2$ auf.

Es hat sich gezeigt, dass Aufnahme- und Verteilungsschichten mit den oben genannten Flächengewichten besonders gute Trageeigenschaften bei einem sehr guten Rücknässeverhalten aufweisen.

Gemäß einer besonders bevorzugten Ausgestaltung der vorliegenden Erfindung ist eine Aufnahme- und Verteilungsschicht so ausgestaltet, dass die Aufnahme- und Verteilungsschicht eine Rücknässung gemessen nach dem EDENA Standard Test: WSP 80.10 (05) von 0,1 bis 0,25 g und ein Flächengewicht nach DIN EN 29073-1 von 10 g/m$^2$ bis 15 g/m$^2$ aufweist.

Die Aufnahme- und Verteilungsschicht umfasst erfindungsgemäß mehrere Prägeflächen.

Die Prägeflächen können trilobal, rund, insbesondere oval, elliptisch oder kreisförmig, eckig, insbesondere rechteckig, quadratisch, dreieckig, viereckig, fünfeckig oder sechseckig, sternförmig oder in Form von Mustern ausgebildet sein. Auch können Stege zum Einsatz kommen, gerade und/oder gebogene Stege. Auf einer Aufnahme- und Verteilungsschicht können auch verschiedene Prägeflächen mit unterschiedlichen Geometrien kombiniert werden.

Die Prägeflächen können die mechanischen, haptischen und optischen Eigenschaften der Aufnahme- und Verteilungsschicht verbessern.

Zusätzlich lassen sich Eigenschaften wie beispielsweise die Rücknässungseigenschaften und die mechanischen Eigenschaften durch die Anordnung und die Größe der Prägeflächen steuern.

Bevorzugt werden Aufnahme- und Verteilungsschichten, bei denen jede der Prägeflächen eine Oberfläche von 0,5 mm$^2$ bis 5 mm$^2$, vorzugsweise 2 mm$^2$ bis 4 mm$^2$ aufweist.

In eigenen Untersuchungen hat sich gezeigt, dass hierdurch gute Rücknässungseigenschaften erhalten werden können.

Gemäß einer bevorzugten Ausgestaltung der vorliegenden Erfindung ist die Aufnahme- und Verteilungsschicht so ausgestaltet, dass die Aufnahme- und Verteilungsschicht eine kumulierte Oberfläche der Prägeflächen von 3 % bis 35 %, vorzugsweise 5 % bis 30 %, besonders bevorzugt 10 % bis 25 % der Gesamtoberfläche der Aufnahme- und Verteilungsschicht aufweist.

In einer bevorzugten Ausführungsform sind die Prägeflächen elliptisch ausgestaltet und besitzen eine Länge der Hauptachse von etwa 2,3 mm bis 2,7 mm, bevorzugt 2,45 mm und eine Länge der Nebenachse von etwa 1,9 mm bis 2,3 mm, bevorzugt 2,08 mm, die Prägefläche beträgt etwa 3,8 mm$^2$ bis 4,3 mm$^2$, bevorzugt 4 mm$^2$ und die kumulierte Oberfläche der Prägeflächen auf der Aufnahme- und Verteilungsschicht beträgt zwischen etwa 23% und 27%, bevorzugt 25 %. Jeweils mehrere Prägeflächen sind bevorzugt um eine in weitere, in einer Mitte einer so gebildeten Figur angeordnet. Zum Beispiel sind sechs Prägeflächen hexagonal zueinander angeordnet und eine weitere Prägefläche befindet sich im Zentrum des Hexagons.

In einer weiteren bevorzugten Ausführungsform sind die Prägeflächen trilobal und/oder stäbchenförmig ausgestaltet. Die Prägefläche beträgt beispielsweise etwa 0,850 mm$^2$ bis etwa 1,150mm$^2$, bevorzugt 0,987 mm$^2$ bei einer trilobalen Gestaltung, und etwa 0,550 mm$^2$ bis etwa 0,780 mm$^2$, bevorzugt 0,655 mm$^2$ bei einer stäbchenförmigen Gestaltung und die kumulierte Oberfläche der Prägeflächen auf der Aufnahme- und Verteilungsschicht beträgt vorzugsweise zwischen 15% und 17,5%, bevorzugt 16,2 % bei einer Mischung aus trilobelen und stäbchenförmigen Prägeflächen. Die Prägeflächen sind beispielsweise auf der Aufnahme- und Verteilungsschicht so angeordnet, dass ein hexagonales Muster ausgebildet wird oder ein anderes sich gleichmäßig wiederholendes Muster von annähernd kreisförmig angeordneten Prägeflächen entsteht.

In einer weiteren bevorzugten Ausführungsform sind die Prägeflächen kreisförmig ausgestaltet. Der Durchmesser der der Prägeflächen beträgt etwa 0,875 mm bis etwa 1,155 mm, vorzugsweise 1 mm. Die Prägefläche beträgt vorzugsweise zwischen 0,766 mm$^2$ und 1,334 mm$^2$, bevorzugt 0,785 mm$^2$ und die kumulierte Oberfläche der Prägeflächen auf der Aufnahme- und Verteilungsschicht beträgt bevorzugt zwischen 16,5% und etwa 18,3%, bevorzugt 17,02 %. Die Prägeflächen sind auf der Aufnahme- und Verteilungsschicht beispielsweise so angeordnet, dass ein hexagonales Muster ausgebildet wird, wobei jeweils 12 Prägemuster pro Hexagon angeordnet sind (1 Prägefläche pro Ecke des Hexagons und jeweils eine Prägefläche zwischen zwei Ecken). Andere Muster sind aber ebenfalls möglich.

In einer weiteren bevorzugten Ausführungsform sind die Prägeflächen stäbchenförmig ausgestaltet. Die Dicke des Stäbchens beträgt beispielsweise zwischen 0,4 mm und 0,7 mm, bevorzugt 0,5 mm, und die Länge des Stäbchens beträgt etwa zwischen 1,9 mm und 3,2 mm, bevorzugt etwa 2,4 mm. Die Prägefläche beträgt bevorzugt zwischen etwa 0,76 mm$^2$ und 2,24 mm$^2$, insbesondere etwa 1,146 mm$^2$. Eine kumulierte Oberfläche der Prägeflächen auf der Aufnahme- und Verteilungsschicht beträgt vorzugsweise zwischen 9,5% und etwa 13,5 %, bevorzugt etwa 10,2 %.

Eine bevorzugte Ausgestaltung der vorliegenden Erfindung betrifft eine Aufnahme- und Verteilungsschicht, die trilobale Fasern mit einem zum Beispiel mittels Mikroskop bestimmten Titer von 1 dtex bis 10 dtex, vorzugsweise mit einen Titer von 2 dtex bis 8 dtex, besonders bevorzugt mit einen Titer von 2 dtex bis 5 dtex aufweist.

Ebenfalls bevorzugt ist eine Aufnahme- und Verteilungsschicht, deren trilobale Fasern einen organischen oder anorganischen Füllstoff aufweisen.

Durch die Verwendung von organischen oder anorganischen Füllstoffen in den trilobalen Fasern können verschiedene positive Effekte erreicht werden. Beispielsweise kann durch die Verwendung von preiswerten Füllstoffen der Verbrauch von teureren Materialien verringert und der Preis der Fasern entsprechend reduziert werden. Die Verwendung von Füllstoffen und die Geometrie der verwendeten Füllstoffe kann aber auch beispielsweise Auswirkungen auf die haptischen oder mechanischen Eigenschaften haben.

Weiter bevorzugt sind Aufnahme- und Verteilungsschichten, deren trilobale Fasern Pigmente enthalten, insbesondere TiOz.

Die Verwendung von Pigmenten in den Fasern führt dazu, dass die Fasern optisch ansprechender sind und auch eine höhere Opazität aufweisen. Insbesondere bei dünnen Aufnahme- und Verteilungsschichten ist es bevorzugt, dass weitere mögliche Schichten, die sich unterhalb der Aufnahme- und Verteilungsschicht befinden, nicht durchscheinen. Die Verwendung von entsprechenden Pigmenten kann aber auch das ästhetische Aussehen der Aufnahme- und Verteilungsschicht verbessern, insbesondere wenn die Aufnahme- und Verteilungsschicht farbige Flüssigkeiten aufgenommen hat.

In eigenen Untersuchungen hat es sich gezeigt, dass Aufnahme- und Verteilungsschichten besonders geeignet sind, wenn die Aufnahme- und Verteilungsschicht Filamente in Maschinenrichtung (MD) und Filamente quer zur Maschinenrichtung (CD) beinhaltet, und das Verhältnis zwischen Filamenten in Maschinenrichtung (MD) zu Filamenten quer zur Maschinenrichtung (CD) 1,1 bis 5,0 beträgt.

Bevorzugt sind dabei Aufnahme- und Verteilungsschichten, die eine Spunbond-Schicht umfassen, die aus trilobalen Polypropylen-Fasern besteht.

Diese Aufnahme- und Verteilungsschichten können kalandriert werden und die kostenintensiven Verfahren wie das "air-through bonding" oder "resin bonding" können vermieden werden.

Weiterhin kann es von Vorteil sein, wenn die Aufnahme- und Verteilungsschicht zusätzlich eine Meltblown-Schicht aufweist.

Es ist dann besonders bevorzugt, wenn sich die Meltblown-Schicht zwischen zwei Spunbond-Schichten befindet.

Gemäß einer Ausgestaltung werden Aufnahme- und Verteilungsschichten vorgeschlagen die biologisch abbaubar ist.

Als biologisch abbaubar wird eine Aufnahme- und Verteilungsschicht verstanden, die innerhalb von 12 Wochen in einer Industriekompostierung nach Europäischer Norm EN 13432 zu mindestens 50 Gew.-% abgebaut werden.

Ebenfalls bevorzugt ist eine Aufnahme- und Verteilungsschicht, deren trilobalen Fasern eine Armdicke von 4 bis 10 $\mu$m, vorzugsweise eine Armdicke von 5 bis 9 $\mu$m, insbesondere eine Armdicke von 5 bis 8 $\mu$m aufweisen und/oder eine Armlänge von 10 bis 40 $\mu$m, vorzugsweise eine Armlänge von 12 bis 30 $\mu$m, besonders bevorzugt eine Armlänge von 14 bis 25 $\mu$m aufweisen.

Die Armdicke und die Armlänge der trilobalen Fasern werden mittels eines Mikroskops bestimmt. Dabei werden die trilobalen Fasern in ein geeignetes Kunstharz eingebettet und anschließend quer zur Faserlänge geschnitten. Die Armlänge wird ab dem Zentrum der trilobalen Faser bis zur Spitze des Armes gemessen. Die Armdicke wird auf halber Länge der Armlänge bestimmt.

Überraschenderweise hat sich gezeigt, dass Fasern mit den beschriebenen Armlängen und/oder Armdicken besonders gute Eigenschaften hinsichtlich Flüssigkeitsweiterleitung und Rücknässung aufweisen. Bei geringeren Armlängen und/oder größeren Armdicken ist insbesondere die vorteilhafte geringe Rücknässung, wie sie überraschend festgestellt wurde, nicht gegeben. Zudem wird auch die Herstellung von Vliesen, wie sie in der erfindungsgemäßen Aufnahme- und Verteilungsschicht vorliegen, erschwert, wenn insbesondere die Armdicke von den genannten Werten abweicht.

Bevorzugt besteht ein Vlies einer Aufnahme- und Verteilungsschicht daher aus trilobalen Fasern, bei denen das Verhältnis von Armdicke zu Armlänge im Bereich von 1 : 10 bis 1:1, insbesondere von 1 : 8 bis 1 : 1,5, vorzugsweise von 1 : 5 bis 1 : 2, besonders 1 : 5 bis 1 : 2,5, beispielsweise 1 : 5, 1 : 4, 1 : 3,5 oder 1 : 3 beträgt. Überraschenderweise hat sich gezeigt, dass hier und insbesondere bei einem Bereich von 1 : 10 bis 1 : 2 eine gute Wirkung hinsichtlich der Rücknässung erzielt werden kann.

Insbesondere handelt es sich bei den trilobalen Fasern um schmelzgesponnene Fasern die unter Verwendung von Spinnplatten hergestellt wurden. Die Spinnplatten weisen vorzugsweise trilobale Bohrungen auf, die eine Armdicke von 50 bis 300 $\mu$m, vorzugsweise eine Armdicke von 100 bis 250 $\mu$m, insbesondere eine Armdicke von 150 bis 230 $\mu$m aufweisen und/oder eine Armlänge von 400 bis 900 $\mu$m, vorzugsweise eine Armlänge von 500 bis 800 $\mu$m, besonders bevorzugt eine Armlänge von 520 bis 760 $\mu$m aufweisen.

Es hat sich gezeigt, dass so trilobale Fasern besonders gut hergestellt werden können. Insbesondere bei einem Verhältnis von Armdicke zu Armlänge der Spinnplatten im Bereich von 1 : 10 bis 1:1, insbesondere von 1 : 8 bis 1 : 1,5, vorzugsweise von 1 : 5 bis 1 : 2, besonders 1 : 5 bis 1 : 2,5, beispielsweise 1 : 5, 1 : 4, 1 : 3,5 oder 1 : 3 lassen sich trilobale Fasern zur Herstellung der erfindungsgemäßen Schichten herstellen. Überraschenderweise hat sich gezeigt, dass hier und insbesondere bei einem Bereich von 1 : 10 bis 1 : 2 besonders formstabile trilobale Fasern hergestellt werden können. Formstabil bedeutet in diesem Zusammenhang, dass die erhaltenen Fasern das selbe oder annähernd das selbe Verhältnis Armdicke zu Armlänge aufweisen, wie die Spinnplatten.

Besonders kritisch ist in diesem Zusammenhang die Armdicke. Weisen die Spinnplatten eine deutlich geringere Armdicke auf, so werden unter anderem hohe Scherraten bei der Herstellung auftreten, wodurch das Material nach dem Durchtritt durch die Spinnplatte expandiert. Dies kann zu Spinninstabilitäten führen. Auch ist keine ausreichende Kontrolle mehr über die finale Fasergeometrie gegeben.

Weisen dagegen die Spinnplatten eine zu große Armdicke gegenüber der Armlänge auf, kann ein trilobaler Querschnitt der Faser nicht mehr erreicht werden. Vielmehr weist eine solche Faser dann einen dreieckigen Querschnitt auf, was ein völlig anderes physikalisches Verhalten des resultierenden Vliesstoffes zur Folge hat.

Zudem sind bei den erfindungsgemäßen Verhältnissen solche Fasern herstellbar, deren Armdicke und Armlänge über die Länge der gesamten Faser hinweg annähernd konstant sind, was zu einer guten Eigenschaft der erfindungsgemäßen Aufnahme- und Verteilungsschicht im Hinblick auf Absorptionsfähigkeit, Tragegefühl aber auch Rücknässung führt. So kann beispielsweise ein nicht im Wesentlichen konstanter trilobaler Faserquerschnitt dazu führen, daß dieses vorteilhafte Verhalten partiell nicht mehr gewährleistet werden kann. Genau dies kann durch die erfindungsgemäß bevorzugten Verhältnisse bei der Herstellung und damit auch bei den Fasern vermieden werden.

Auch bevorzugt ist eine Aufnahme- und Verteilungsschicht, die eine Luftdurchlässigkeit nach DIN EN ISO 9237 (12-1995) (Fläche des Messkopfes 20 cm$^2$, Prüfdruck 200 Pa) von 4000 bis 9500 L/m$^2$s, vorzugsweise von 5000 bis 9000 L/m$^2$s aufweist.

Darüber hinaus ist es bevorzugt, dass die Aufnahme- und Verteilungsschicht eine Dicke von 0,3 bis 2,0 mm, vorzugsweise eine Dicke von 0,3 bis 1,5 mm, besonders bevorzugt eine Dicke von 0,4 bis 1,0 mm, ganz besonders bevorzugt 0,4 bis 0,6 mm aufweist.

Die in dieser Beschreibung angegebenen Ausgestaltungen der Aufnahme- und Verteilungsschicht können beliebig miteinander kombiniert werden. Der Fachmann kann durch Testversuche die gewünschten Eigenschaften der Aufnahme- und Verteilungsschicht einstellen, indem er die einzelnen Parameter variiert.

Es hat sich in eigenen Versuchen gezeigt, dass insbesondere eine Aufnahme- und Verteilungsschicht für eine aufzunehmende Flüssigkeit bevorzugt ist, die aus einem Vlies besteht, das zumindest trilobale Fasern umfasst, vorzugsweise daraus besteht, wobei die Aufnahme- und Verteilungsschicht eine Rücknässung gemessen nach dem EDENA Standard Test: WSP 80.10 (05) von 0,1 g bis 0,25 g aufweist, die Aufnahme- und Verteilungsschicht hydrophil ist, wobei die trilobalen Fasern der Aufnahme- und Verteilungsschicht aus Polypropylen, vorzugsweise aus isotaktischem Polypropylen bestehen, wobei die Aufnahme- und Verteilungsschicht ein Flächengewicht nach DIN EN 29073-1 von 10 g/m$^2$ bis 17 g/m$^2$ aufweist, wobei die Aufnahme- und Verteilungsschicht mehrere Prägeflächen umfasst, die eine Oberfläche von jeweils 2 mm$^2$ bis 5 mm$^2$ aufweisen und die Aufnahme- und Verteilungsschicht eine kumulierte Oberfläche der Prägeflächen von 3 % bis 32 % der Gesamtoberfläche der Aufnahme- und Verteilungsschicht aufweist, die trilobale Fasern einen Titer von 3 dtex bis 6 dtex aufweisen, wobei die Aufnahme- und Verteilungsschicht beispielsweise zwei Spunbond-Schichten und eine Meltblown-Schicht aufweist, wobei die Meltblown-Schicht zwischen den zwei Spunbond-Schichten angeordnet ist, und wobei die Aufnahme- und Verteilungsschicht eine Dicke von 0,4 mm bis 0,6 mm aufweist.

Darüber hinaus ist es besonders bevorzugt, dass das Vlies der Aufnahme- und Verteilungsschicht vollständig aus trilobalen Fasern besteht und die Aufnahme- und Verteilungsschicht eine um den Faktor 1:2, vorzugsweise um den Faktor 1:5, besonders bevorzugt um den Faktor 1:8 geringere Rücknässung aufweist, als eine Aufnahme- und Verteilungsschicht, die unter identischen Bedingungen hergestellt wurde, aber deren Vlies vollständig aus runden Fasern mit gleichem Titer wie die trilobalen Fasern besteht. Sofern also eine Aufnahme- und Verteilungsschicht, deren Vlies vollständig aus runden Fasern besteht, eine Rücknässung von Beispielsweise 2 g aufweist, sollte die Rücknässung einer Aufnahme- und Verteilungsschicht, deren Vlies vollständig aus trilobalen Fasern besteht, geringer als 1 g (Faktor 1:2), vorzugsweise geringer als 0,4 g (Faktor 1:5), besonders bevorzugt geringer als 0,25 (Faktor 1:8) sein.

Insbesondere ist es dabei bevorzugt, dass beide verglichenen Aufnahme- und Verteilungsschichten

- identische Prägeflächen aufweisen, vorzugsweise wie weiter oben bezüglich der Prägeflächenoberfläche, der Prägeflächengeometrie und der kumulierten Oberfläche der Prägeflächen als bevorzugt angegeben,
- identische Flächengewichte aufweisen, vorzugsweise wie weiter oben als bevorzugt angegeben,
- eine identische Dicke der Aufnahme- und Verteilungsschicht, vorzugsweise wie weiter oben als bevorzugt angegeben, aufweisen

und die Fasern aus identischem Material, vorzugsweise wie weiter oben als bevorzugt angegeben, hergestellt sind, wobei sämtliche Werte und Eigenschaften unter gleichen Bedingungen ermittelt werden.

Ein weiterer Aspekt im Zusammenhang mit der vorliegenden Erfindung betrifft ein Flächengebilde zumindest bestehend aus:

- einer beschriebenen Aufnahme- und Verteilungsschicht und
- einer Absorptionsschicht.

Bevorzugt ist ein Flächengebilde, das zusätzlich ein Backsheet umfasst, wobei sich die Absorptionsschicht zwischen dem Backsheet und der Aufnahme- und Verteilungsschicht befindet.

Weiter bevorzugt ist ein Flächengebilde, das keine weitere Schicht auf der Aufnahme- und Verteilungsschicht aufweist. Beispielsweise kann ein Topsheet beziehungsweise dessen Funktion Bestandteil der Aufnahme- und Verteilungsschicht sein.

Ein weiterer Aspekt der vorliegenden Erfindung betrifft einen Hygieneartikel zumindest bestehend aus einer Aufnahme- und Verteilungsschicht wie vorgeschlagen.

Bevorzugt ist ein Hygieneartikel, bei dem der Hygieneartikel ein Artikel ist ausgewählt aus der Gruppe bestehend aus Windeln, Inkontinenzeinlagen, Slipeinlagen, Damenbinden und Kosmetikpads.

Weiter bevorzugt ist ein Hygieneartikel, bei dem die vorgeschlagene Aufnahme- und Verteilungsschicht so angebracht ist, dass sie beim üblichen Gebrauch direkten Kontakt mit der Haut aufweist, also keine weitere Oberschicht zum Bespiel in Form eines Topsheet aufweist.

Da sich die Aufnahme- und Verteilungsschicht aufgrund der sehr guten mechanischen, optischen und haptischen Eigenschaften auch eignet um direkten Kontakt mit der Haut zu haben, kann auf eine zusätzlich Schicht als Oberschicht beziehungsweise Deckschicht, beispielsweise Topsheet verzichtet werden. Hierdurch ist es möglich, dass entweder die Dicke des Hygieneartikels reduziert wird oder bei gleicher Dicke die Effizienz des Hygieneartikels verbessert wird, zum Beispiel verglichen mit Hygieneartikeln, die zusätzlich eine Oberschicht auf der Aufnahme- und Verteilungsschicht aufweisen.

Durch das Einsparen einer zusätzlichen Schicht kann aber auch der Herstellungsprozess von Hygieneartikeln vereinfacht werden und damit auch die Herstellungskosten und die Produktkosten gesenkt werden. Zusätzlich kann auch das Packmaß der fertigen Hygieneartikel reduziert werden.

Die der vorliegenden Erfindung zugrundeliegende Aufgabe wird in einer weiteren Ausführungsform gelöst durch die Verwendung von trilobalen Fasern in einer Aufnahme- und Verteilungsschicht zur Reduzierung oder Verhinderung der Rücknässung. Überraschenderweise hat sich gezeigt, dass Aufnahme- und Verteilungsschichten Flüssigkeiten sehr gut aufnehmen und eine geringe Durchdringungszeit aufweisen, gleichzeitig aber auch eine sehr geringe Rücknässung aufweisen, wenn sie trilobale Fasern umfassen und insbesondere daraus bestehen. Die Verwendung der trilobalen Fasern ermöglicht, dass ein Rückschlagen der Flüssigkeit verhindert wird. Dieses Verhalten ist mit üblichen Rundfasern, wie sie im Stand der Technik beschrieben sind, nicht zu erzielen und war so auch nicht zu erwarten.

Ein bevorzugter Aspekt der vorliegenden Erfindung betrifft ein Verfahren zur Herstellung eines Flächengebildes umfassend die folgenden Schritte:

- Bereitstellen einer beschriebenen Aufnahme- und Verteilungsschicht
- Kontaktieren mit einer Absorptionsschicht.

Weitere Aspekte im Zusammenhang mit der vorliegenden Erfindung betreffen vorteilhafte Verwendungen der beschriebenen Aufnahme- und Verteilungsschicht zur Herstellung von:

- Hygieneartikeln, insbesondere Windeln, Inkontinenzeinlagen, Slipeinlagen, Damenbinden, Kosmetikpads,
- Putztücher, Wischtücher, Wischmopptücher
- Filtern beispielsweise für Gase, Aerosole und Flüssigkeiten,
- Wundverbänden, Wundkompressen
- Dämmmaterialen, Akustikvliesstoffen
- Einlagestoffen
- Dachunterspannbahnen,
- Geovliesen, oder von
- Abdeckungen für die Feld- und Gemüsewirtschaft.

Weitere vorteilhafte Ausgestaltungen und Weiterbildungen sind in den nachfolgenden Figuren angegeben. Die daraus hervorgehenden jeweiligen Merkmale sind jedoch nicht auf einzelne Figuren oder Ausgestaltungen beschränkt. Vielmehr können ein oder mehrere Merkmale der obigen Beschreibung zusätzlich zu Weiterbildungen kombiniert werden.

Es zeigen:

Fig. 1: eine schematische Darstellung einer Aufnahme- und Verteilungsschicht;

Fig. 2: eine schematische Darstellung einer Aufnahme- und Verteilungsschicht, die eine Meltblown-Schicht und zwei Spunbond-Schichten aufweist;

Fig. 3a:    eine schematische Darstellung einer Aufnahme- und Verteilungsschicht;

Fig. 3b:    eine schematische Darstellung einer Aufnahme- und Verteilungsschicht unter Einwirkung eines Druckes;

Fig. 4:    eine schematische Darstellung eines Flächengebildes mit einer Aufnahme- und Verteilungsschicht und einer Absorptionsschicht;

Fig. 5:    eine schematische Darstellung eines Flächengebildes mit einer Aufnahme- und Verteilungsschicht und einer Absorptionsschicht unter Einwirkung von Wasser;

Fig. 6:    Mikroskop-Aufnahmen von trilobalen Fasern;

Fig. 7:    einen schematischen Aufbau einer Düse zur Herstellung von trilobalen Fasern,

Fig. 8:    einen schematischen Aufbau einer Düse zur Herstellung von trilobalen Fasern,

Fig. 9    eine graphische Darstellung von den in den Beispielen ermittelten Durchdringungszeiten (Strike-through time; SST) für die Vergleichsbeispiele 2a und 2b und für Beispiel 2,

Fig. 10    eine graphische Darstellung von den in den Beispielen ermittelten Durchdringungszeiten (Strike-through time; SST) für die Vergleichsbeispiele 1a und 1b und für Beispiel 1,

Fig. 11    eine graphische Darstellung von den in den Beispielen ermittelten Rücknässungswerten (rewet) für die Vergleichsbeispiele 2a und 2b und für Beispiel 2 und

Fig. 12    eine graphische Darstellung von den in den Beispielen ermittelten Rücknässungswerten (rewet) für die Vergleichsbeispiele 1a und 1b und für Beispiel 1.

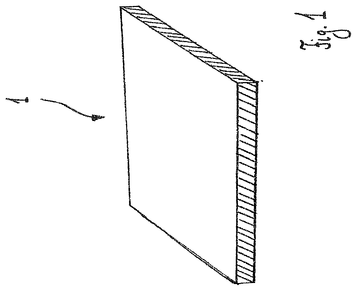

Figur 1 zeigt schematisch einen Ausschnitt einer beschriebenen Aufnahme- und Verteilungsschicht 1.

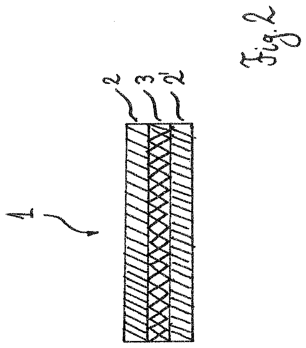

Figur 2 zeigt schematisch den Aufbau einer beschriebenen Aufnahme- und Verteilungsschicht 1, die eine Meltblown-Schicht 3 und zwei Spunbond-Schichten aufweist, wobei sich die Meltblown-Schicht 3 zwischen den zwei Spunbold-Schichten 2, 2' befindet. Die gesamte Anordnung stellte eine beschriebenen Aufnahme- und Verteilungsschicht 1 dar.

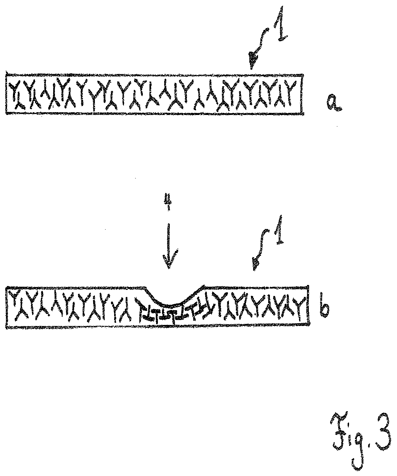

Figur 3a stellt unter schematisch eine beschriebenen Aufnahme- und Verteilungsschicht 1 dar. Figur 3b zeigt schematisch eine beschriebene Aufnahme- und Verteilungsschicht 1 unter Einwirkung eines Druckes 4. Die trilobalen Fasern der beschriebenen Aufnahme- und Verteilungsschicht 1 werden unter Einwirkung des Druckes 4 verformt, zusammengedrückt und bilden eine kompakte Schicht.

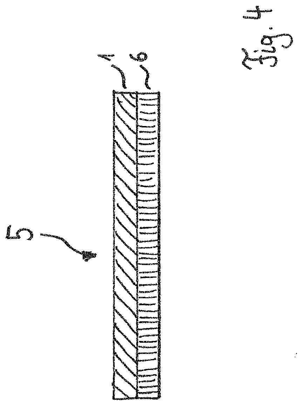

Figur 4 stellt schematische ein beschriebenes Flächengebilde 5 dar, das eine beschriebene Aufnahme- und Verteilungsschicht 1 und eine Absorptionsschicht 6 umfasst.

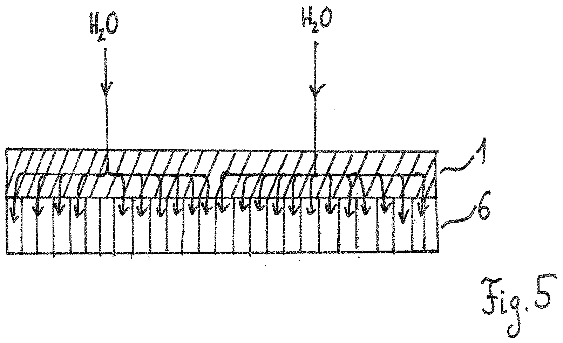

Die Abbildung in Figur 5 stellt schematische ein Flächengebilde 5 mit einer Aufnahme- und Verteilungsschicht 1 und einer Absorptionsschicht 6 unter Einwirkung von einer Flüssigkeit wie Wasser $H_2O$ dar. Das Wasser $H_2O$ wird von der Aufnahme- und Verteilungsschicht 1 aufgenommen und in der Aufnahme- und Verteilungsschicht 1 verteilt, bevor es anschließend an die Absorptionsschicht 6 abgegeben wird.

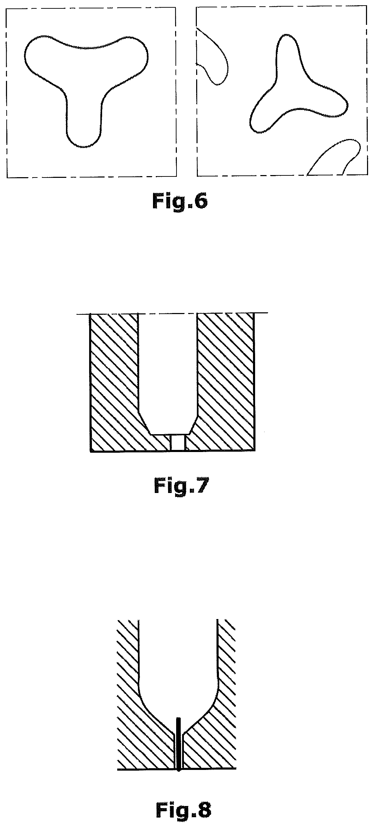

Figur 6 zeigt Mikroskopaufnahmen von trilobalen Fasern einer Aufnahme- und Verteilungsschicht 1.

Figur 7 zeigt schematisch einen beispielsweisen Aufbau einer Düse zur Herstellung von trilobalen Fasern.

Figur 8 zeigt schematisch einen beispielsweisen Aufbau einer Düse zur Herstellung von trilobalen Fasern.

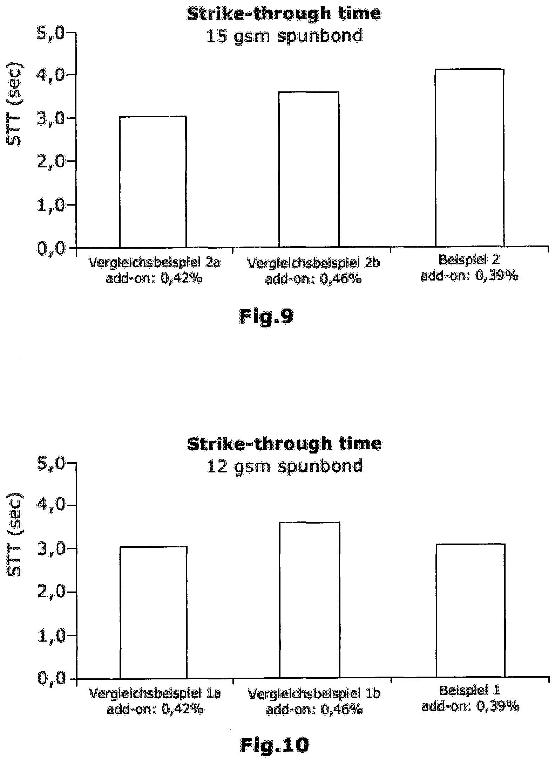

Figur 9 zeigt in einem Balkendiagramm die in den Beispielen ermittelten Durchdringungszeiten (Strike-through time; SST) für die Vergleichsbeispiele 2a und 2b und für Beispiel 2. Die Vliese (spunbond) weisen ein Flächengewicht von 15 g/cm$^2$ (15 gsm) auf.

Figur 10 zeigt in einem Balkendiagramm die in den Beispielen ermittelten Durchdringungszeiten (Strike-through time; SST) für die Vergleichsbeispiele 1a und 1b und für Beispiel 1. Die Vliese (spunbond) weisen ein Flächengewicht von 12 g/cm$^2$ (12 gsm) auf.

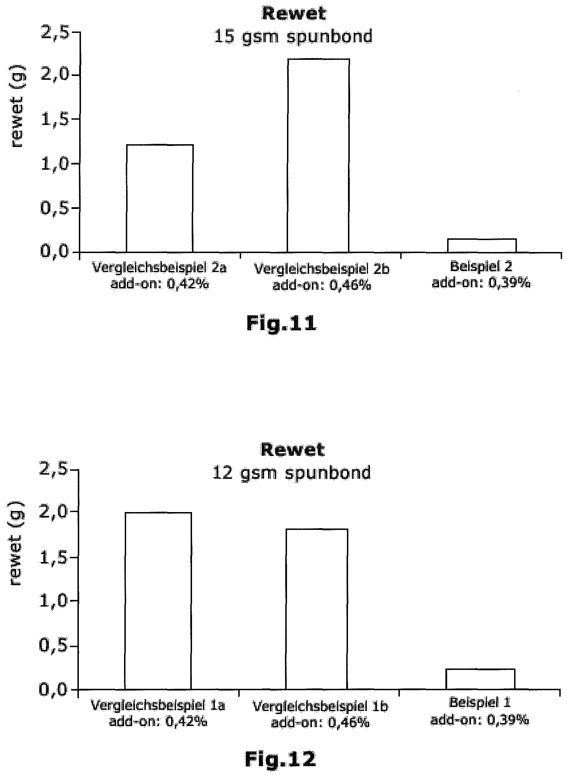

Figur 11 zeigt in einem Balkendiagramm die in den Beispielen ermittelten Rücknässungswerte (rewet) für die Vergleichsbeispiele 2a und 2b und für Beispiel 2. Die Vliese (spunbond) weisen ein Flächengewicht von 15 g/cm$^2$ (15 gsm) auf.

Figur 12 zeigt in einem Balkendiagramm die in den Beispielen ermittelten Rücknässungswerte (rewet) für die Vergleichsbeispiele 1a und 1b und für Beispiel 1. Die Vliese (spunbond) weisen ein Flächengewicht von 12 g/cm$^2$ (12 gsm) auf.

**Messmethoden**

Sämtliche nachfolgenden Bestimmungen wurden, wenn nicht anders angegeben oder zweckdienlich, bei 23 °C, 1013 mbar und 50 % rel. Luftfeuchtigkeit durchgeführt. Die Proben wurden vor dem Vermessen für 24 Stunden unter Laborbedingungen (23 °C und 50 % rel. Luftfeuchtigkeit) gelagert.

**Bestimmung des Filamenttiters**

Die Ermittlung des Filamenttiters erfolgt mittels eines Mikroskops. Die Umrechnung des gemessenen Titers (in Mikrometern) in Dezitex erfolgt nach folgender Formel (Dichte PP = 0,91 g/cm$^3$):

$$\left(\frac{Titer_{\mu m}}{2}\right)^2 \cdot \pi \cdot \rho \left[\frac{g}{cm^3}\right] \cdot 0{,}01 = Titer_{dtex} \left[\frac{g}{10^4 m}\right]$$

**Bestimmung des Flächengewichtes**

Die Flächengewichtsbestimmung erfolgt nach DIN EN 29073-1 an 10 x 10 cm großen Probekörpern. Die Vliesstoffdicke wird gemessen als Abstand zweier planparalleler Messflächen bestimmter Größe, zwischen denen sich die Vliesstoffe unter einem vorgegebenen Messdruck befinden. Die Methode wird analog der DIN EN ISO 9073-2 ausgeführt. Auflagegewicht 125 g, Messfläche 25 cm$^2$, Messdruck 5 g/cm$^2$

**Bestimmung der Luftdurchlässigkeit**

Die Messung der Luftdurchlässigkeit erfolgt gemäß DIN EN ISO 9237. Die Fläche des Messkopfes beträgt 20 cm$^2$, der angelegte Prüfdruck 200 Pa.

**Bestimmung der Durchdringungszeit**

Die Messung der Durchdringungszeiten der Vliesstoffe ("Liquid strike-through time") erfolgt nach dem EDENA Standard Test: WSP 70.3 (05) ("Standard Test Method for Nonwoven Coverstock Liquid Strike-Through Time Using Simulated Urine").

**Rücknässung ("rewet" oder "wetback")**

Die Messung der Rücknässung der Vliesstoffe ("Liquid strike-through time") erfolgt nach dem EDENA Standard Test: WSP 80.10 (05) ("Standard Test Method for Nonwovens Coverstock Wetback").

**Beispiel 1**

Es wurde ein Vliesstoff mit einem Flächengewicht von 12 g/m$^2$ und einem Titer von 2 dtex hergestellt, wobei die Fasern eine trilobale Struktur aufweisen. Die Fasern bestanden aus einem kommerziell erhältlichen Ziegler-Natta-
**[0107]** Polypropylen. Der Add-On des Vlieses wurde anschließend auf ca. 0,4 % eingestellt (ermittelt mittels Extraktion mit Isopropanol; Genauigkeit +/-0,03 %). Von dem hergestellten Vliesstoff wurden anschließend die Rücknässung und die Durchdringungszeit bestimmt.

**Beispiel 2**

Analog zu Beispiel 1 wurde ein Vliesstoff mit einem Flächengewicht von 15 g/m$^2$ und einem Titer von 2 dtex hergestellt, wobei die Fasern eine trilobale Struktur aufweisen. Die Fasern bestanden aus einem kommerziell erhältlichen Ziegler-Natta-Polypropylen. Der Add-On des Vlieses wurde anschließend auf ca. 0,4 % eingestellt (ermittelt mittels Extraktion mit Isopropanol; Genauigkeit +/- 0,03%). Von dem hergestellten Vliesstoff wurden anschließend die Rücknässung und die Durchdringungszeit bestimmt.

**Vergleichsbeispiel 1a**

Analog zu Beispiel 1 wurde ein Vliesstoff mit einem Flächengewicht von 12 g/m$^2$ und einem Titer von 2 dtex hergestellt, wobei die Fasern eine runde Struktur aufweisen. Die Fasern bestanden aus einem kommerziell erhältlichen Ziegler-Natta-Polypropylen. Der Add-On des Vlieses wurde anschließend auf ca. 0,4 % eingestellt (ermittelt mittels Extraktion mit Isopropanol; Genauigkeit +/- 0,03 %). Von dem hergestellten Vliesstoff wurden anschließend die Rücknässung und die Durchdringungszeit bestimmt.

**Vergleichsbeispiel 1b**

Analog zu Beispiel 1 wurde ein Vliesstoff mit einem Flächengewicht von 12 g/m$^2$ und einem Titer von 2 dtex hergestellt, wobei die Fasern eine runde Struktur aufweisen. Die Fasern bestanden aus einem kommerziell erhältlichen Metallocen-Polypropylen. Der Add-On des Vlieses wurde anschließend auf ca. 0,4 % eingestellt (ermittelt mittels Extraktion mit Isopropanol; Genauigkeit +/- 0,03 %). Von dem hergestellten Vliesstoff wurden anschließend die Rücknässung und die Durchdringungszeit bestimmt.

**Vergleichsbeispiel 2a**

Analog zu Beispiel 1 wurde ein Vliesstoff mit einem Flächengewicht von 15 g/m$^2$ und einem Titer von 2 dtex hergestellt, wobei die Fasern eine runde Struktur aufweisen. Die Fasern bestanden aus einem kommerziell erhältlichen Ziegler-Natta-Polypropylen. Der Add-On des Vlieses wurde anschließend auf ca. 0,4 % eingestellt (ermittelt mittels Extraktion mit Isopropanol; Genauigkeit +/- 0,03 %). Von dem hergestellten Vliesstoff wurden anschließend die Rücknässung und die Durchdringungszeit bestimmt.

**Vergleichsbeispiel 2b**

Analog zu Beispiel 1 wurde ein Vliesstoff mit einem Flächengewicht von 15 g/m$^2$ und einem Titer von 2 dtex hergestellt, wobei die Fasern eine runde Struktur aufweisen. Die Fasern bestanden aus einem kommerziell erhältlichen Metallocen-Polypropylen. Der Add-On des Vlieses wurde anschließend auf ca. 0,4 % eingestellt (ermittelt mittels Extraktion mit Isopropanol; Genauigkeit +/- 0,03%). Von dem hergestellten Vliesstoff wurden anschließend die Rücknässung und die Durchdringungszeit bestimmt.

Nachfolgend werden die ermittelten Werte der Rücknässung und der Durchdringungszeit sowie die eingestellten Add-On-Werte für die Beispiele und Vergleichsbeispiele tabellarisch wiedergegeben:

|  | Durchdringungszeit [s] | Rücknässung [g] | Add-On |
|---|---|---|---|
| Beispiel 1 | 3,02 ±0,13 | 0,20 ±0,10 | 0,39 % |
| Beispiel 2 | 4,04 ±1,00 | 0,13 f0,03 | 0,39 % |
| Vergleichsbeispiel 1a | 3,09 f0,33 | 2,00 f0,93 | 0,42 % |
| Vergleichsbeispiel 1b | 3,53 ±0,72 | 1,80 ±0,44 | 0,46 % |
| Vergleichsbeispiel 2a | 3,01 ±0,29 | 1,21 ±0,26 | 0,42 % |
| Vergleichsbeispiel 2b | 3,57 f0,28 | 2,30 f0,99 | 0,46 % |

Bei den Beispielen 1 und 2 ist deutlich zu erkennen, dass die Rücknässung um ein Vielfaches geringer ist, als bei den Vergleichsbeispielen 1a bis 2b. Zur Veranschaulichung wurden die Ergebnisse in den Figuren 9 bis 12 graphisch dargestellt.

**Patentansprüche**

**1.** Aufnahme- und Verteilungsschicht für eine aufzunehmende Flüssigkeit umfassend wenigstens ein Vlies, das aus trilobalen Fasern besteht, wobei das Vlies ein Spinnvlies ist,

wobei das Flächengewicht der Aufnahme- und Verteilungsschicht 5 g/m$^2$ bis 20 g/m$^2$ beträgt,
wobei die Aufnahme- und Verteilungsschicht mehrere Prägeflächen umfasst, und wobei die Aufnahme- und Verteilungsschicht eine Rücknässung gemessen nach dem EDENA Standard Test: WSP 80.10 (05) von 0,01 g bis 0,50 g, vorzugsweise 0,05 g bis 0,3 g, besonders bevorzugt 0,1 g bis 0,25 g aufweist.

2. Aufnahme- und Verteilungsschicht nach Anspruch 1, wobei die trilobalen Fasern der Aufnahme- und Verteilungsschicht aus einem Polyolefin, insbesondere Polypropylen oder Polyethylen, Polylactide oder aus Copolymeren oder aus Mischungen davon im Wesentlichen bestehen.

3. Aufnahme- und Verteilungsschicht nach Anspruch 1 oder 2, **dadurch gekennzeichnet, dass** die trilobalen Fasern eine Armdicke von 4 $\mu$m bis 10 $\mu$m, bevorzugt von 5 $\mu$m bis 9$\mu$m, und/oder eine Armlänge von 10 $\mu$m bis 40 $\mu$m, bevorzugt von 12 $\mu$m bis 30 $\mu$m.

4. Aufnahme- und Verteilungsschicht nach einem der Ansprüche 1 bis 3, **dadurch gekennzeichnet, dass** das Verhältnis von Armdicke zu Armlänge 1:10 bis 1:1, insbesondere 1:8 bis 1:1,5, bevorzugt 1:5 bis 1:2,5 beträgt.

5. Aufnahme- und Verteilungsschicht nach Anspruch 1, wobei jeder der Prägefläche eine Oberfläche von 0,5 mm$^2$ bis 5 mm$^2$, vorzugsweise von 2 mm$^2$ bis 4 mm$^2$ aufweist und/oder die Aufnahme- und Verteilungsschicht eine kumulierte Oberfläche der Prägeflächen von 3 % bis 35 %, vorzugsweise 5 % bis 30 %, besonders bevorzugt 10 % bis 25 % der Gesamtoberfläche der Aufnahme- und Verteilungsschicht aufweist.

6. Aufnahme- und Verteilungsschicht nach einem der vorherigen Ansprüche, wobei die Aufnahme- und Verteilungsschicht zwei Spunbond-Schichten und eine Meltblown-Schicht umfasst und die Meltblown-Schicht zwischen den zwei Spunbond-Schichten angeordnet ist.

7. Aufnahme- und Verteilungsschicht nach einem der vorherigen Ansprüche, wobei die Aufnahme- und Verteilungsschicht eine Dicke von 0,3 mm bis 2,0 mm, vorzugsweise eine Dicke von 0,3 mm bis 1,5 mm, besonders bevorzugt eine Dicke von 0,4 mm bis 1,0 mm, ganz besonders bevorzugt 0,4 mm bis 0,6 mm aufweist.

8. Aufnahme- und Verteilungsschicht nach einem der vorherigen Ansprüche, wobei das Vlies vollständig aus trilobalen Fasern besteht und die Aufnahme- und Verteilungsschicht eine geringere Rücknässung aufweist, als eine Aufnahme- und Verteilungsschicht, die unter identischen Bedingungen hergestellt wurde, aber deren Vlies vollständig aus runden Fasern mit gleichem Titer wie die trilobalen Fasern besteht.

9. Flächengebilde zumindest bestehend aus:

   - einer Aufnahme- und Verteilungsschicht nach einem der Ansprüche 1 bis 8 und
   - einer Absorptionsschicht.

10. Hygieneartikel zumindest bestehend aus einer Aufnahme- und Verteilungsschicht nach einem der Ansprüche 1 bis 8.

11. Verwendung eines Vlieses, das aus trilobalen Fasern besteht, in der Aufnahme- und Verteilungsschicht, gemäß einem der Ansprüche 1 bis 8 zur Reduzierung oder Verhinderung der Rücknässung.

**Claims**

1. An absorbing and distributing layer for a liquid to be absorbed, comprising at least one non-woven consisting of trilobal fibers, said non-woven being a spunbonded fabric,

   wherein the mass per unit area of said absorbing and distributing layer is from 5 g/m$^2$ to 20 g/m$^2$,
   wherein said absorbing and distributing layer comprises several embossing surfaces, and
   wherein said absorbing and distributing layer has a rewetting as measured by the EDENA standard test WSP 80.10 (05) from 0.01 g to 0.50 g, preferably from 0.05 g to 0.3 g, more preferably from 0.1 g to 0.25 g.

2. The absorbing and distributing layer according to claim 1, wherein the trilobal fibers of the absorbing and distributing layer are essentially made of a polyolefin, especially polypropylene or polyethylene, polylactides or copolymers or mixtures thereof.

3. The absorbing and distributing layer according to claim 1 or 2, **characterized in that** said trilobal fibers have an arm thickness of 4 $\mu$m to 10 $\mu$m, preferably from 5 $\mu$m to 9 $\mu$m, and/or an arm length of 10 $\mu$m to 40 $\mu$m, preferably of 12 $\mu$m to 30 $\mu$m.

4. The absorbing and distributing layer according to any of claims 1 to 3, **characterized in that** the ratio of arm thickness to arm length is from 1:10 to 1:1, especially from 1:8 to 1:1.5, preferably from 1:5 to 1:2.5.

5. The absorbing and distributing layer according to claim 1, wherein each of the embossing surfaces has a surface area of 0.5 mm$^2$ to 5 mm$^2$, preferably 2 mm$^2$ to 4 mm$^2$, and/or the absorbing and distributing layer has a cumulated surface area of the embossing surfaces of 3% to 35%, preferably 5% to 30%, more preferably 10% to 25%, of the total surface area of the absorbing and distributing layer.

6. The absorbing and distributing layer according to any of the precedent claims, wherein said absorbing and distributing layer comprises two spunbond layers and one melt-blown layer, the melt-blown layer being arranged between the two spunbond layers.

7. The absorbing and distributing layer according to any of the precedent claims, wherein said absorbing and distributing layer has a thickness of 0.3 to 2.0 mm, preferably a thickness of 0.3 to 1.5 mm, more preferably a thickness of 0.4 to 1.0 mm, even more preferably of 0.4 to 0.6 mm.

8. The absorbing and distributing layer according to any of the precedent claims, wherein said non-woven completely consists of trilobal fibers, and the absorbing and distributing layer has a lower rewetting as compared to an absorbing and distributing layer manufactured under identical conditions, but whose non-woven completely consists of round fibers with the same titer as the trilobal fibers.

9. A sheet material at least consisting of:

   - an absorbing and distributing layer according to any of claims 1 to 8; and
   - an absorption layer.

10. A personal hygiene product at least consisting of an absorbing and distributing layer according to any of claims 1 to 8.

11. Use of a non-woven consisting of trilobal fibers in the absorbing and distributing layer according to any of claims 1 to 8 for reducing or preventing rewetting.

**Revendications**

1. Couche de distribution et d'absorption pour un liquide à recevoir, comprenant un matériau non tissé consistant en fibres trilobées, ledit matériau non tissé étant un non tissé filé-lié,

   dans laquelle le grammage de ladite couche de distribution d'acquisition est de 5 g/m$^2$ à 20 g/m$^2$,
   dans laquelle ladite couche de distribution d'acquisition comprend plusieurs surfaces d'estampage, et
   dans laquelle ladite couche de distribution d'acquisition présente un remouillage - mesuré selon le test standard EDENA WSP 80.10 (05) - de 0,01 g à 0,50 g, de préférence de 0,05 g à 0,3 g, de préférence encore de 0,1 g à 0,25 g.

2. Couche de distribution et d'absorption selon la revendication 1, dans laquelle lesdites fibres trilobées de la couche de distribution et d'absorption consistent essentiellement en une polyoléfine, notamment polypropylène ou polyéthylène, polylactide, ou des copolymères ou mélanges de ceux-ci.

3. Couche de distribution et d'absorption selon la revendication 1 ou 2, **caractérisée en ce que** lesdites fibres trilobées présentent une épaisseur de lobe de 4 $\mu$m à 10 $\mu$m, de préférence de 5 $\mu$m à 9 $\mu$m, et/ou une longueur de lobe de 10 $\mu$m à 40 $\mu$m, de préférence de 12 $\mu$m à 30 $\mu$m.

4. Couche de distribution et d'absorption selon l'une quelconque des revendications 1 à 3, **caractérisée en ce que** le rapport de l'épaisseur de lobe à la longueur de lobe est de 1 : 10 à 1 : 1, notamment de 1 : 8 à 1 : 1,5, de préférence de 1 : 5 à 1 : 2,5.

**5.** Couche de distribution et d'absorption selon la revendication 1, dans laquelle chacune des surfaces d'estampage présente une aire de 0,5 mm$^2$ à 5 mm$^2$, de préférence de 2 mm$^2$ à 4 mm$^2$, et/ou ladite couche de distribution et d'absorption présente une aire accumulée des surfaces d'estampage de 3 % à 35 %, de préférence de 5 % à 30 %, de préférence encore de 10 % à 25 %, de la surface totale de la couche de distribution et d'absorption.

**6.** Couche de distribution et d'absorption selon l'une quelconque des revendications précédentes, dans laquelle ladite couche de distribution et d'absorption comprend deux couches filées-liées et une couche d'extrusion-soufflage, et ladite couche d'extrusion-soufflage est disposée entre les deux couches filées-liées.

**7.** Couche de distribution et d'absorption selon l'une quelconque des revendications précédentes, dans laquelle ladite couche de distribution et d'absorption présente une épaisseur de 0,3 mm à 2,0 mm, de préférence une épaisseur de 0,3 mm à 1,5 mm, de préférence encore une épaisseur de 0,4 mm à 1,0 mm, de manière tout particulièrement préférée de 0,4 mm à 0,6 mm.

**8.** Couche de distribution et d'absorption selon l'une quelconque des revendications précédentes, dans laquelle ledit matériau non tissé consiste entièrement de fibres trilobées, et ladite couche de distribution et d'absorption présente un remouillage inférieur à celui d'une couche de distribution et d'absorption préparée sous des conditions identiques, mais dont le matériau non tissé consiste entièrement de fibres rondes ayant le même titre que lesdites fibres trilobées.

**9.** Structure de surface constituée au moins par :

- une couche de distribution et d'absorption selon l'une quelconque des revendications 1 à 8, et
- une couche d'absorption.

**10.** Article de toilette, constitué au moins par une couche de distribution et d'absorption selon l'une quelconque des revendications 1 à 8.

**11.** Utilisation d'un matériau non tissé consistant en fibres trilobées dans la couche de distribution et d'absorption selon l'une quelconque des revendications 1 à 8 pour la réduction ou prévention du remouillage.

Fig. 1

Fig. 2

Fig. 3

Fig. 4

Fig. 5

**Fig.6**

**Fig.7**

**Fig.8**

Fig.9

Fig.10

**Rewet**
15 gsm spunbond

**Fig.11**

**Rewet**
12 gsm spunbond

**Fig.12**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5314743 A
- EP 0547498 A1
- US 2010310845 A1

**In der Beschreibung aufgeführte Nicht-Patentliteratur**

- **P. K. CHATTERJEE.** Absorbency. Elsevier, 1985